# EUROPEAN PATENT SPECIFICATION

(11) **EP 4 687 895 B1**
(45) Date of publication and mention of the grant of the patent: **09.12.2026**
(21) Application number: 24718132.4
(22) Date of filing: 05.04.2024
(51) Int. Cl.: A61K 31/436, A61K 9/00, A61K 9/10, A61K 9/14, A61P 27/02

(54) **USE OF PIMECROLIMUS FOR THE TREATMENT OF DRY EYE IN PATIENTS CHARACTERIZED BY A CORNEAL STAINING SCORE OF 4 OR 5 ON THE OXFORD GRADING SCALE**
VERWENDUNG VON PIMECROLIMUS ZUR BEHANDLUNG DES TROCKENEN AUGES BEI PATIENTEN MIT EINEM HORNHAUTFÄRBUNGSWERT VON 4 ODER 5 AUF DER OXFORD-ABSTUFUNG
UTILISATION DU PIMECROLIMUS POUR LE TRAITEMENT DE L'OEIL SEC CHEZ DES PATIENTS CARACTÉRISÉS PAR UN SCORE DE COLORATION CORNÉENNE DE 4 OU 5 SUR L'ÉCHELLE DE CLASSEMENT OXIDOMYCOLE

(30) Priority: 06.04.2023 EP 23167035
(43) Date of publication of application: 11.02.2026
(73) Proprietor: Premark Pharma GmbH, 4153 Reinach (CH)
(72) Inventor: VESSEY, Ian, 4153 Reinach (CH); MAYER, Friedrich K., 4104 Oberwil (CH)
(74) Representative: Vossius & Partner Patentanwälte Rechtsanwälte mbB
(86) International application number: PCT/EP2024/059309
(87) International publication number: WO 2024/209038

(56) References cited:
- WO-A1-2020/234389
- LEONARDI ANDREA ET AL: "Efficacy and Safety of 0.1% Cyclosporine a Cationic Emulsion in the Treatment of Severe Dry Eye Disease: A Multicenter Randomized Trial", EUROPEAN JOURNAL OF OPHTHALMOLOGY, vol. 26, no. 4, 7 April 2016 (2016-04-07), IT, pages 287 - 296, XP093223411, ISSN: 1120-6721, Retrieved from the Internet <URL:https://journals.sagepub.com/doi/full-xml/10.5301/ejo.5000779> DOI: 10.5301/ejo.5000779
- OFRI R ET AL: "Clinical evaluation of pimecrolimus eye drops for treatment of canine keratoconjunctivitis sicca: A comparison with cyclosporine A", VETERINARY JOURNAL, BAILLIERE TINDALL, LONDON, GB, vol. 179, no. 1, 1 January 2009 (2009-01-01), pages 70 - 77, XP025740629, ISSN: 1090-0233, [retrieved on 20071022], DOI: 10.1016/J.TVJL.2007.08.034
- NELL BARBARA ET AL: "The effect of topical pimecrolimus on keratoconjunctivitis sicca and chronic superficial keratitis in dogs: results from an exploratory study", VETERINARY OPHTHALMOLOGY, BLACKWELL SCIENCE, OXFORD, GB, vol. 8, no. 1, 1 January 2005 (2005-01-01), pages 39 - 46, XP002527017, ISSN: 1463-5224, DOI: 10.1111/J.1463-5224.2005.04062.X
- YINGFANG FAN ET AL: "Pimecrolimus micelle exhibits excellent therapeutic effect forKeratoconjunctivitis Sicca", COLLOIDS AND SURFACES B: BIOINTERFACES, ELSEVIER AMSTERDAM, NL, vol. 140, 19 December 2015 (2015-12-19), pages 1 - 10, XP029413102, ISSN: 0927-7765, DOI: 10.1016/J.COLSURFB.2015.11.059
- AUW-H� DRICH C ET AL: "Behandlung der chronischen Blepharokeratokonjunktivitis mit lokalen Kalzineurininhibitoren", DER OPHTHALMOLOGE, SPRINGER, BERLIN, DE, vol. 106, no. 7, 6 June 2009 (2009-06-06), pages 635 - 638, XP019739215, ISSN: 1433-0423, DOI: 10.1007/S00347-009-1954-9
- YUKAKO OGAWA ET AL: "/ SUPPLEMENT S99 VISUAL FUNCTION QUESTIONNAIRE ASSESSMENT USING NEI- VFQ-25 IN KERATOCONUS PATIENTS", 1 January 2005 (2005-01-01), XP055638524, Retrieved from the Internet <URL:https://www.sciencedirect.com/science/article/pii/S1542012412705189/pdfft?md5=25d48168ce87e9fe17c06192bd854fe1&pid=1-s2.0-S1542012412705189-main.pdf>

## Description

### BACKGROUND INFORMATION

Keratoconjunctivitis sicca (KCS), commonly referred to as dry eye, is a disease involving the ocular surface, tear film, and related ocular tissues. Dry eye is a multifactorial disease that results in symptoms of discomfort, visual disturbance, and tear film instability with potential damage to the ocular surface. It is accompanied by increased osmolarity of the tear film and inflammation of the ocular surface.

Dry eye affects millions of people and accounts for 17% to 25% of patient visits to ophthalmologists.

Dry eye is associated with a broad spectrum of ocular symptoms including irritation, burning, itching, pain, gritty feeling, foreign body sensation, ocular fatigue, photophobia and vision disturbances. Patients seek treatment in order to manage their ocular symptoms. Alleviation of ocular discomfort is the primary goal of treatment. Ulceration and perforation of the cornea may occur in the most severe cases.

Additionally, a number of clinical signs are used to diagnose and assess dry eye, including corneal staining and tear break up time. Corneal fluorescein staining qualifies and quantifies the viability of the epithelium. Punctate epithelial erosions are a common feature of dry eye disease. Damage to the cornea manifests as small epithelial erosions which stain with fluorescein. These can be scattered over the ocular surface and sometimes become larger, confluent areas. Thereby, the density and extent of corneal staining enables assessment of disease severity and monitoring of response to treatment.

The various manifestations of dry eye have recently been classified into 2 broad categories:
1. Aqueous Deficient (deficiency in tear production, often associated with Sjögren's syndrome)
2. Evaporative (mainly due to deficiencies in the lipid layer of the tear film, resulting from meibomian gland dysfunction (MGD), blepharitis, lid abnormalities, or the wearing of contact lenses).

Research suggests deficiency in tear production may result from a cytokine-mediated inflammatory process affecting both the lacrimal glands and the ocular surface. Meibomian gland dysfunction is also often associated with inflammation and resolving this may also improve gland function and treat evaporative dry eye.

Current standard of care for dry eye is palliative therapy with artificial tears / ocular lubricants or punctal occlusion. Additionally, there are 4 marketed ophthalmic products containing the calcineurin inhibitor ciclosporin.

Restasis^{™} (0.05% ciclosporin ophthalmic emulsion) is currently marketed in the United States and is indicated 'to increase tear production in patients whose tear production is presumed to be suppressed due to ocular inflammation associated with keratoconjunctivitis sicca'. However, Phase 3 regulatory studies failed to demonstrate improvement in ocular symptoms compared to vehicle control and the product is not approved to treat dry eye symptoms.

Cequa^{™} (0.09% ciclosporin ophthalmic solution) is currently marketed in the United States and is indicated 'to increase tear production in patients with keratoconjunctivitis sicca'. Phase 3 regulatory studies failed to demonstrate improvement in ocular symptoms compared to vehicle control and the product is not approved to treat dry eye symptoms.

Vevye^{™} (0.1% ciclosporin ophthalmic solution) achieved marketing authorization from the FDA for the treatment of dry eye, but in the pivotal Phase 3 study, failed to demonstrate an improvement in ocular symptoms (dry eye score) compared to vehicle control.

In Europe, Ikervis^{™} (1 mg/mL (0.1%) ciclosporin emulsion) is indicated for the treatment of severe keratitis in adult patients with dry eye disease, which has not improved despite treatment with tear substitutes. In Phase 3 regulatory studies no significant improvement of symptoms was observed for Ikervis compared to placebo/vehicle control after 6 months of treatment. Leonardi Andrea et al (DOI: 10.5301/ejo.5000779) discloses that eye drops comprising cyclosporin A (CsA), a calcineurin inhibitor, are effective in treating patients with severe dry eye diseases (grade 4 on Oxford scale, measured with corneal fluorescein staining). 22.7% of patients receiving CsA for 6 months demonstrated an improvement of ≥2 grades in Oxford scale and 30% in VAS (Visual Analogue Scale assessment of ocular discomfort). The same improvement was observed in 20.9% of patients receiving vehicle (p value=0.744). 39.6% of both groups showed a ≥30% improvement in OSDI (Ocular Surface Disease Index questionnaire) (p value= 0.939). However, when stringent criteria were applied, i.e. when the group of responders having an improvement of ≥3 grades instead of 2 in CFS was studies, the results showed a statistically significant improvement in the CsA-treated group compared to the vehicle-treated one (p=0.016). Moreover, Leonardi et al support that there is a weak correlation between signs and symptoms in dry eye disease, which might explain the poor statistical significance observed in the study.

The ascomycin macrolactam derivative pimecrolimus is a calcineurin inhibitor and shares the known mode of action of ciclosporin and tacrolimus. Pimecrolimus is a potent anti-inflammatory agent which blocks transcription of inflammatory cytokines. It binds with high affinity to macrophilin-12 and inhibits the Ca⁺²-dependent phosphatase calcineurin. Pimecrolimus down-regulates the production of Th1 (IL-2, INFy) and Th2 (IL-4, IL-5, IL-10)-type cytokines in T-cells, and inhibits the proliferation of T-cells after antigen-specific stimulation. In addition, pimecrolimus prevents the release of cytokines and pro-inflammatory mediators from mast cells in vitro after stimulation by immunoglobulin E (IgE)/antigen.

A randomized, single center, double-masked, placebo/vehicle-controlled clinical study assessed the safety and efficacy (using a Controlled Adverse Environment model) of pimecrolimus ocular suspension (0.1%, 0.3% and 1.0% b.i.d.) in the treatment of mild to moderate KCS. These formulations were proven to be safe over a 12-week treatment period. The results of this study suggest efficacy of pimecrolimus on corneal staining upon topical ocular application. However, no improvement across treatment groups could be detected with regard to relief of ocular discomfort and alleviation of dry eye symptoms compared to placebo/vehicle control.

There is therefore a need for pharmacological treatments for dry eye that improve both signs ***and*** symptoms. Ocular discomfort is the primary reason that dry eye patients consult an eye care professional and relief of symptoms is a primary goal of treatment.

One objective of the present invention is to provide a treatment that is effective in the treatment of dry eye, in particular in the treatment of ocular discomfort and other symptoms associated with dry eye.

Another objective of the present invention is to identify a group of dry eye patients that respond particularly well to a treatment with pimecrolimus.

### SUMMARY OF THE INVENTION

The present invention is characterized in the herein provided embodiments and claims.

Any references in the description to methods of treatment refer to the compounds, pharmaceutical compositions and medicaments of the present invention for use in a method for treatment of the human (or animal) body by therapy. In particular, the present invention relates, *inter alia,* to the following embodiments:
1. A composition comprising pimecrolimus for use in the treatment of severe keratoconjunctivitis sicca (dry eye).
2. The composition for use according to embodiment 1, wherein keratoconjunctivitis sicca (dry eye) is characterized by a corneal staining score of 4 or 5 on the Oxford grading scale.
3. The composition for use of embodiment 1 or 2, wherein the composition comprises between 0.1% (wt/wt) and 2% (wt/wt) pimecrolimus.
4. The composition for use of any one of embodiments 1 to 3 comprising 0.3% (wt/wt) or 1% (wt/wt) pimecrolimus.
5. The composition for use of any one of embodiments 1 to 4, wherein the composition is a suspension, a solution or a semi-solid, in particular wherein the semi-solid is an ointment, a cream, a gel, or a paste.
6. The composition for use according to any one of embodiments 1 to 5, wherein the suspension is a water-based suspension, a glycerol-based suspension or a water-glycerol-based suspension.
7. The composition for use according to embodiment 5 or 6, wherein the suspension further comprises one or more of a wetting agent, a suspending agent, an osmotic agent, a preservative and/or a pH-adjusting agent.
8. The composition according to any one of embodiments 5 to 7, wherein the suspension comprises a carbomer.
9. The composition for use of any one of embodiments 1 to 5, wherein the ointment comprises a medium-chain triglyceride and a microcrystalline wax.
10. The composition for use of embodiment 9, wherein the content of the medium-chain triglyceride is between 30% (wt/wt) and 60% (wt/wt), in particular wherein the content of the medium-chain triglyceride is 50% (wt/wt).
11. The composition for use of embodiment 9 or 10, wherein the content of the microcrystalline wax is between 3% (wt/wt) and 10% (wt/wt), in particular wherein the content of the microcrystalline wax is 6% (wt/wt).
12. The composition for use of any one of embodiments 9 to 11, wherein the composition is an ointment comprising 0.3% (wt/wt) pimecrolimus, 50% (wt/wt) medium-chain triglyceride and 6% (wt/wt) microcrystalline wax; or
   wherein the composition is an ointment comprising 0.1% (wt/wt) pimecrolimus, 50% (wt/wt) medium-chain triglyceride, 6% (wt/wt) microcrystalline wax; or
   wherein the composition is an ointment comprising 1% (wt/wt) pimecrolimus, 50% (wt/wt) medium-chain triglyceride, 6% (wt/wt) microcrystalline wax.
13. The composition for use of any one of embodiments 9 to 12, wherein the medium-chain triglyceride comprises caprylic acid and/or capric acid.
14. The composition for use of any one of embodiments 9 to 13 further comprising a preservative.
15. The composition for use of embodiment 14, wherein the content of the preservative is between 0.5% (wt/wt) and 2% (wt/wt), in particular wherein the content of the preservative is 1.0% (wt/wt).
16. The composition for use of embodiment 14 or 15, wherein the preservative is phenyl ethyl alcohol.
17. The composition for use of any one of embodiments 1 to 16, wherein the treatment period is between 2 weeks and 48 weeks, in particular wherein the treatment period is 12 weeks or 24 weeks.
18. The composition for use of any one of embodiments 1 to 16, wherein the composition is administered continuously or administered as a chronic intermittent treatment.
19. The composition for use of any one of embodiments 1 to 18, wherein at the end of the treatment, the subjects show at least a 0.3 score unit greater improvement (Oxford scale) from baseline compared to placebo/vehicle control for the mean score for corneal staining and/or at least 5 mm greater improvement (on a 100mm visual analogue scale (VAS)) from baseline compared to placebo/vehicle control for the mean score for ocular discomfort.
20. The composition for use of any one of embodiments 1 to 19, wherein a unit dose of the composition is administered once, twice, three times, four times a day.

Accordingly, in a particular embodiment, the invention relates to a composition comprising pimecrolimus for use in the treatment of severe keratoconjunctivitis sicca (dry eye).

It has been surprisingly shown by the inventors that pimecrolimus is effective in the treatment of severe dry eye and especially in the treatment of symptoms of severe dry eye. Specifically, in a severe dry eye population, administration of pimecrolimus was shown to result in greater improvements from baseline in mean scores for corneal staining (Oxford Scale) (**Tables 3 and** 5) and ocular discomfort (**Tables 4 and 6**) than administration of a placebo/vehicle control. Notably, statistically significant improvements in ocular discomfort compared to placebo/vehicle control were only achieved in patients with a corneal staining score of 4 or 5 on the Oxford scale.

Surprisingly, none of the currently marketed ocular formulations of a calcineurin inhibitor; Restasis^{™}, Cequa^{™}, Vevye^{™} and Ikervis^{™} have demonstrated, in Phase 3 regulatory studies, an ability to improve the ocular symptoms associated with dry eye or severe keratitis compared to a placebo/vehicle control.

Within the present invention, dry eye patients are preferably characterized by a corneal staining score of at least 4 on the Oxford grading scale. The Oxford grading scale divides corneal staining into six groups according to severity from 0 (absent) to 5 (severe). The examiner compares the overall appearance of the patient's corneal staining with a reference figure, simulating the pattern of staining encountered in dry eye disease. The skilled person is capable of determining a patient's corneal staining score. For additional information on the Oxford grading scale, Bron et al. (Cornea, 2003, 22(7):640-50) may be consulted.

It has been shown by the inventors that pimecrolimus is particularly effective in the treatment of severe dry eye, as characterized by a corneal staining score of 4 or 5 on the Oxford grading scale. In this severe dry eye population, even greater improvements from baseline for the mean scores for corneal staining (Oxford scale) (**Table 5**) and especially ocular discomfort (**Table 6**) were observed when compared to a placebo/vehicle control. Thus, in a particularly preferred embodiment, the invention relates to the composition for use according to the invention, wherein severe keratoconjunctivitis sicca (dry eye) is characterized by a corneal staining score of 4 or 5 on the Oxford grading scale.

In a particular embodiment, the invention relates to a composition comprising pimecrolimus for use in the treatment of keratoconjunctivitis sicca (dry eye) in a subject, wherein the subject is characterized by a corneal staining score of 4 or 5 on the Oxford grading scale.

In a particular embodiment, the invention relates to a composition comprising pimecrolimus for use in the treatment of keratoconjunctivitis sicca (dry eye) in a human subject, wherein the human subject is characterized by a corneal staining score of 4 or 5 on the Oxford grading scale.

In addition to corneal staining, further criteria may be used to identify patients with dry eye. For example, dry eye patients may be characterized by an ocular discomfort score of at least 40% (40 mm) on a 100 mm visual analog scale (VAS). The severity of ocular discomfort is illustrated on a visual analog scale ranging from 0 mm (no ocular discomfort) to 100 mm (the most severe ocular discomfort). Suitable questionnaires to determine the severity of ocular discomfort are known in that art and are available to the skilled person (see *e.g.,* Klimek et al., 2017, Allergo J Int, 26(1), p.16-24). Unless indicated otherwise, the term visual analog scale used herein always refers to a 100mm visual analog scale.

In certain embodiments, dry eye is characterized by an ocular discomfort score of at least 40 mm, at least 50 mm or at least 60 mm on a visual analog scale ranging from 0 mm (no ocular discomfort) to 100 mm (severe ocular discomfort).

In certain embodiments, severe dry eye is characterized by a corneal staining score of at least 4 on the Oxford grading scale and an ocular discomfort score of at least 40 mm on a visual analog scale ranging from 0 mm (no ocular discomfort) to 100 mm (severe ocular discomfort). In certain embodiments, severe dry eye is characterized by a corneal staining score of at least 4 on the Oxford grading scale and an ocular discomfort score of at least 50 mm, or at least 60 mm on a visual analog scale ranging from 0 mm (no ocular discomfort) to 100 mm (severe ocular discomfort).

In addition to corneal staining and/or ocular discomfort, patients may further be identified as severe dry eye patients based on the results of a Schirmer's test. Schirmer's test determines whether the eye produces enough tears to keep it moist. This test is used when a person experiences very dry eyes or excessive watering of the eyes. The test works by the principle of capillary action, which allows the water in tears to travel along the length of a paper test strip in an identical fashion as a horizontal capillary tube. The rate of travel along the test strip is positively correlated to the rate of tear production. A negative (more than 10 mm of moisture on the filter paper in 5 minutes) test result is normal.

In certain embodiments, dry eye is characterized by a Schirmer's test score of 7 mm or less in 5 minutes (≤7 mm/5 minutes), 6 mm or less in 5 minutes (≤6 mm/5 minutes) or 5 mm or less in 5 minutes (≤5 mm/5 minutes).

In certain embodiments, severe dry eye is characterized (i) by a corneal staining score of at least 4 on the Oxford grading scale and (ii) an ocular discomfort score of at least 40 mm on a visual analog scale ranging from 0 mm (no ocular discomfort) to 100 mm (severe ocular discomfort) and/or a Schirmer's test score of 7 mm or less in 5 minutes (≤7 mm/5 minutes).

In certain embodiments, severe dry eye is characterized (i) by a corneal staining score of at least 4 on the Oxford grading scale and (ii) an ocular discomfort score of at least 50 mm on a visual analog scale ranging from 0 mm (no ocular discomfort) to 100 mm (severe ocular discomfort) and/or a Schirmer's test score of 6 mm or less in 5 minutes (≤6 mm/5 minutes).

In certain embodiments, severe dry eye is characterized (i) by a corneal staining score of at least 4 on the Oxford grading scale and (ii) an ocular discomfort score of at least 60 mm on a visual analog scale ranging from 0 mm (no ocular discomfort) to 100 mm (severe ocular discomfort) and/or a Schirmer's test score of 5 mm or less in 5 minutes (≤5 mm/5 minutes).

The composition according to the invention may comprise any concentration of pimecrolimus that is suitable for effectively treating severe dry eye. The concentration of pimecrolimus in the composition may depend on various factors, such as, without limitation, the amount of the composition that is administered per time unit, the formulation of the composition and the route of administration. However, the skilled person is capable of identifying an effective concentration of pimecrolimus.

In a particular embodiment, the invention relates to the composition for use according to the invention, wherein the composition comprises between 0.1% (wt/wt) and 10% (wt/wt) pimecrolimus.

The active compound of the compositions and methods of this invention is pimecrolimus. Pimecrolimus is an immunomodulating agent of the calcineurin inhibitor class used in the treatment of atopic dermatitis (eczema). It is available as a topical cream, once marketed by Novartis under the trade name Elidel^{®}. The preparation of pimecrolimus and pharmaceutical compositions containing pimecrolimus are disclosed in International Publications Nos. WO 01/60345 A2, WO 97/03654, WO 99/01458 and WO 01/90110 A1, U.S. Patents No: 6,197,781 and 6,004,973 and in EP patents 1 754 482 A1, 1482 936 B1 and 0 427 680 B1.

Pimecrolimus, an ascomycin macrolactam derivative, is a proven anti-inflammatory agent, which blocks transcription of "early" cytokines. The rationale for use of pimecrolimus in patients with dry eye is to treat the disease by targeting the associated inflammation. Pimecrolimus has been shown to be highly potent in vitro and highly effective in animal models of skin inflammation. In vivo data indicate that topical pimecrolimus has greater anti-inflammatory activity in the skin than ciclosporin, but a lower potential for systemic immunosuppression than ciclosporin and tacrolimus. In contrast to topical corticosteroids, topical pimecrolimus does not induce skin atrophy and there is no evidence its ocular administration is associated with cataracts or raised intraocular pressure.

In certain embodiments, the composition for use according to the invention comprises between 0.1% (wt/wt) and 10% (wt/wt) pimecrolimus. In certain embodiments, the composition for use according to the invention comprises between 0.1% (wt/wt) and 9% (wt/wt) pimecrolimus. In certain embodiments, the composition for use according to the invention comprises between 0.1% (wt/wt) and 8% (wt/wt) pimecrolimus. In certain embodiments, the composition for use according to the invention comprises between 0.1% (wt/wt) and 7% (wt/wt) pimecrolimus. In certain embodiments, the composition for use according to the invention comprises between 0.1% (wt/wt) and 6% (wt/wt) pimecrolimus. In certain embodiments, the composition for use according to the invention comprises between 0.1% (wt/wt) and 5% (wt/wt) pimecrolimus. In certain embodiments, the composition for use according to the invention comprises between 0.1% (wt/wt) and 4% (wt/wt) pimecrolimus. In certain embodiments, the composition for use according to the invention comprises between 0.1% (wt/wt) and 3% (wt/wt) pimecrolimus. In a particularly preferred embodiment, the composition for use according to the invention comprises between 0.1% (wt/wt) and 2% (wt/wt) pimecrolimus.

In certain embodiments, the composition for use according to the invention comprises about 0.1% (wt/wt) pimecrolimus, about 0.2% (wt/wt) pimecrolimus, about 0.3% (wt/wt) pimecrolimus, about 0.4% (wt/wt) pimecrolimus, about 0.5% (wt/wt) pimecrolimus, about 0.6% (wt/wt) pimecrolimus, about 0.7% (wt/wt) pimecrolimus, about 0.8% (wt/wt) pimecrolimus, about 0.9% (wt/wt) pimecrolimus, about 1.0% (wt/wt) pimecrolimus, about 1.1% (wt/wt) pimecrolimus, about 1.2% (wt/wt) pimecrolimus, about 1.3% (wt/wt) pimecrolimus, about 1.4% (wt/wt) pimecrolimus, about 1.5% (wt/wt) pimecrolimus, about 1.6% (wt/wt) pimecrolimus, about 1.7% (wt/wt) pimecrolimus, about 1.8% (wt/wt) pimecrolimus, about 1.9% (wt/wt) pimecrolimus or about 2.0% (wt/wt) pimecrolimus.

In a particularly preferred embodiment, the composition for use according to the invention comprises about 0.3% (wt/wt) pimecrolimus. In another particularly preferred embodiment, the composition for use according to the invention comprises about 1% (wt/wt) pimecrolimus.

Besides the active ingredient pimecrolimus, the composition according to the invention may comprise one or more excipients. Information on the properties, specifications and characteristics of the excipients are described e.g. in standard texts such as Fiedler, H.P.; 1996; Lexikon der Hilfsstoffe für Pharmazie, Kosmetik und angrenzende Gebiete; Editio Cantor Verlag Aulendorf (Germany), and Kibbe, A.H.; 2000; Handbook of Pharmaceutical Excipients, a joint publication of Pharmaceutical Press, London (UK), and American Pharmaceutical Association, Washington (US) as well as manufacturers' brochures. Preferably, all excipients used in the composition of the invention follow the US and/or European Pharmacopeia requirements.

As used herein, "treatment" (and grammatical variations thereof such as "treat" or "treating") refers to clinical intervention in an attempt to alter the natural course of the subject being treated, and can be performed either for prophylaxis or for treatment of the signs and symptoms of dry eye. Desirable effects of treatment include, but are not limited to, preventing occurrence or recurrence of disease, alleviation of signs and symptoms, diminishment of any direct or indirect pathological consequences of the disease, decreasing the rate of disease progression, amelioration or palliation of the disease state, and remission or improved prognosis. Within the present invention, the composition is preferably used for the treatment of dry eye as defined above.

The term "subject" or the plural form "subjects" as used herein denote any animal, preferably a mammal, and more preferably a human. Examples of subjects include humans, non-human primates, rodents, guinea pigs, rabbits, sheep, pigs, goats, cattle, cows, horses, dogs and cats. Within the present invention, the terms "subject" and "patient" are used interchangeably. It is to be understood that the composition for use according to the invention or the method according to the invention may be used for the treatment of a single subject suffering from dry eye, as well as for the treatment of groups of subjects suffering from dry eye.

The term "composition", as used herein, refers to a composition comprising at least one pharmaceutically active ingredient and one or more pharmaceutically acceptable excipients. Herein, the pharmaceutically active ingredient, which is pimecrolimus, provides the therapeutic effect for the treatment of a disease in a subject or a group of subjects.

The composition may be formulated in any way that is suitable for the treatment of dry eye, preferably by topical application. In a particular embodiment, the invention relates to the composition for use according to the invention, wherein the composition is a suspension, a solution or a semi-solid formulation, such as an ointment, a cream, a gel, or a paste.

The term "composition", as used herein, refers to a composition comprising at least one pharmaceutically active ingredient and one or more pharmaceutically acceptable excipients. Herein, the pharmaceutically active ingredient, which is pimecrolimus, provides the therapeutic effect for the treatment of a disease in a subject or a group of subjects. The terms "formulation" and "composition" are intended to have the same meaning and are used interchangeably throughout this specification.

In certain embodiments, the composition of the invention is formulated as a suspension. The advantage of a suspension is that it can be delivered to the eye as a drop. Another advantage of the suspension is the ability to be able to have the pH close to the physiological pH of the eye for comfort. In addition, the suspension formulation of pimecrolimus is extremely stable, even during stress tests and autoclaving. Pimecrolimus delivered as a suspension allows for sufficient residence time in the cul-de-sac of the eye to treat the signs and symptoms of dry eye. This suspension formulation of pimecrolimus may be prepared in a non-sterile environment and may then be sterilized in bulk by autoclaving without affecting the overall stability of the formulation. Finally, it is intended that all excipients used in this formulation meet US and European pharmacopoeia requirements.

The term "suspension" as used herein refers to a system in which small solid particles are essentially uniformly dispersed in a liquid medium. That is, pimecrolimus is present in the suspension in the form of small solid particles. Preferably, pimecrolimus is present in micronized form. By the term "micronized", as used herein, is meant the standard usage in the art that the drug particles are processed, for example by milling, bashing and/or grinding, to significantly reduce particle size over those produced naturally during chemical synthesis. Suitably for use herein, at least 50% of the particles are 30 micron or less, suitably at least 50% of the particles are 10 micron or less, suitably at least 50% of the particles are 5 micron or less.

The particles may be suspended in any suitable liquid. In certain embodiments, the particles may be suspended in water or glycerol, or a mixture thereof. Thus, in a particular embodiment, the invention relates to the composition for use according to the invention, wherein the suspension is a water-based suspension or a glycerol-based suspension, or a water-glycerol-based suspension.

The suspension may comprise additional non-pharmaceutically active ingredients, such as a wetting agent, a suspending agent, an osmotic agent, a preservative and/or agents to adjust the pH to a defined value.

The term "wetting agent" as used herein is intended to mean a compound used to aid in attaining intimate contact between solid particles and liquids. Exemplary wetting agents include, by way of example and without limitation, gelatin, casein, lecithin (phosphatides), gum acacia, cholesterol, polysaccharides and substituted polysaccharides and mixtures thereof (such as tragacanth or carrageenan), stearic acid, benzalkonium chloride, calcium stearate, glycerol monostearate, cetostearyl alcohol, cetomacrogol emulsifying wax, sorbitan esters, polyoxyethylene alkyl ethers (e.g., macrogol ethers such as cetomacrogol 1000), polyoxyethylene castor oil derivatives, polyoxyethylene sorbitan fatty acid esters, (e.g., TWEEN^{™}s), polyethylene glycols, polyoxyethylene stearates colloidal silicon dioxide, phosphates, sodium dodecylsulfate, carboxymethylcellulose calcium, carboxymethylcellulose sodium, methylcellulose, hydroxyethylcellulose, hydroxyl propylcellulose, hydroxypropylmethylcellulose phthalate, noncrystalline cellulose, magnesium aluminum silicate, triethanolamine, polyvinyl alcohol, and polyvinylpyrrolidone (PVP). Tyloxapol (a nonionic liquid polymer of the alkyl aryl polyether alcohol type, also known as superinone or triton) is another useful wetting agent, combinations thereof and other such materials known to those of ordinary skill in the art.

In certain embodiments, the wetting agent is a poloxamer. The term "poloxamer" as used herein encompasses block polymers of a lipophilic (e.g., polypropylene glycol) and two hydrophilic moieties (e.g., polyethylene glycol) derived by copolymerization of propylene oxide and ethylene oxide. The known poloxamers are usually referred to as "poloxamer" in conjunction with a number. In a particularly, preferred embodiment, the wetting agent is poloxamer 407.

The term "suspending agent" as used herein refers to a pharmaceutical acceptable excipient that promotes particle suspension or dispersion and reduces sedimentation. Suspending agents retard settling and agglomeration of particles by functioning as an energy barrier, which minimizes interparticle attraction. Suspending agents include protective colloids and viscosity-inducing agents. Protective colloids differ from surfactants in that they do not reduce interfacial tension. Many agents that are protective colloids in low concentration (<0.1%) are viscosity builders in higher concentrations (>0.1%). See G. Jain, R. K. Khar, F. J. Ahmad, Theory and Practice of Physical Pharmacy, 2011. The increase in viscosity of the solution is helpful to prevent sedimentation of the suspended particles. A suspension may have well developed thixotropy: At rest the solution is sufficiently viscous to prevent sedimentation and thus aggregation or caking of the particles. When agitation is applied the viscosity is reduced and provide good flow characteristic.

Examples of suspending agents include polysaccharides, inorganic salts, and polymers. Specific examples of suspending agents include, without limitation, alginates, methylcellulose, hydroxyethylcellulose, carboxymethylcellulose, colloidal silicon dioxide, agar, calcium stearate, hypromellose, magnesium aluminum silicate, guar gum, carboxymethylcellulose sodium, microcrystalline cellulose, acacia, tragacanth, xanthan gum, bentonite, carbomer, carageenan, powdered cellulose, gelatin, polyethylene glycol, povidone, dextrin, medium-chain triglycerides, sucrose, hydroxypropyl methyl cellulose, chitosan, polyoxyethylene, polyoxy-propylene ethers and combinations thereof. See Handbook of pharmaceutical excipients, 6th, Raymond C Rowe, Paul J Sheskey and Marian E Quinn 2009.

In a particularly preferred embodiment, the suspending agent is a carbomer. The term "carbomer", as used herein, is a generic (i.e. nonproprietary) name adopted by USP-NF, United States Adopted Names Council (USAN) and CTFA/PCPC for various Carbopol^{™} polymers. In a particularly preferred embodiment, the carbomer is a carbomer that is suitable for use in pharmaceutical suspensions. In certain embodiments, the carbomer is a type B carbomer (viscosity of 25,000 - 45,000 mPa·s when provided as 0.5% gel, pH 7.3-7.8, 25°C). In certain embodiments, the carbomer is Carbopol 934P or Carbopol 974P. Thus, in a particular embodiment, the invention relates to the composition for use according to the invention, wherein the suspension further comprises a Carbopol/carbomer.

The term "osmotic agent" as used herein includes all pharmaceutically acceptable inert water-soluble compounds suitable for inducing osmosis as referred to in, for example, the Pharmacopoeias, Hagers Handbuch der Pharmazeutischen Praxis: Für Apotheker, Ärzte, Drogisten und Medizinalbeamte, and Remington's Pharmaceutical Sciences. Examples of compounds suitable as osmotic agents include water soluble salts of inorganic acids such as magnesium chloride or magnesium sulfate, lithium chloride, sodium chloride, potassium chloride, lithium hydrogen phosphate, sodium hydrogen phosphate, potassium hydrogen phosphate, lithium dihydrogen phosphate, sodium dihydrogen phosphate, and potassium dihydrogen phosphate; water soluble salts of organic acids such as sodium acetate, potassium acetate, magnesium succinate, sodium benzoate, sodium citrate, and sodium ascorbate; nonionic organic compounds with high water solubility, e.g., carbohydrates such as mannitol, sorbitol, arabinose, ribose, xylose, glucose, fructose, mannose, galactose, sucrose, maltose, lactose, and raffinose; water-soluble amino acids such as glycine, leucine, alanine, and methionine; urea and urea derivatives; and the like and mixtures thereof.

In another embodiment, the invention relates to a composition for use according to the invention further comprising a preservative.

A preservative is a substance or a chemical that is added to products such as food, beverages, pharmaceutical drugs, paints, biological samples, cosmetics, wood, and many other products to prevent decomposition by microbial growth or by undesirable chemical changes.

The composition of the present invention may further comprise any preservative in any amount, provided that the composition retains its claimed technical effect in the presence of the preservative. The preservative may be any preservative, preferably an ophthalmologically acceptable preservative. Suitable preservatives include
(a) a quaternary ammonium compound such as e.g. benzalkonium chloride (N-benzyl-N-(C8-C18-alkyl)-N,N-dimethylammonium chloride), benzoxonium chloride, benzethonium chloride, cetrimide (hexadecyl-trimethylammonium bromide), sepazonium chloride, cetylpyridinium chloride, domiphen bromide (Bradosol^{®}) or the like,
(b) alkyl-mercury salts of thiosalicylic acid, such as e.g. thiomersal, phenylmercuric nitrate, phenylmercuric acetate or phenylmercuric borate,
(c) parabens, such as e.g. methylparaben or propylparaben,
(d) alcohols, such as e.g. chlorobutanol, benzyl alcohol or phenyl ethyl alcohol,
(e) biguanide derivatives, such as e.g. chlorohexidine or polyhexamethylene biguanide,
(f) sodium perborate,
(g) imidazolidinyl urea as known and commercially available under the trade name Germal^{®}II,
(h) sorbic acid,
(i) stabilized oxychloro complexes such as known and commercially available under the trade name Purite^{®},
(k) polyglycol-polyamine condensation resins, such as known and commercially available e.g. under the trade name Polyquart^{®} from Henkel KGaA,
(I) stabilized hydrogen peroxide generated from a source of hydrogen peroxide for providing an effective trace amount of resultant hydrogen peroxide, e.g. sodium perborate tetrahydrate, and/or
(m) a mixture of any components (a) to (I).

Preferably, the preservative comprised in the composition according to the invention is benzalkonium chloride. In certain embodiments, the suspension of the present invention comprises 0.015% benzalkonium chloride.

The term "pH-adjusting agent," as used herein, refers to an agent used to maintain the pH in the desired range. Suitable examples of pH-adjusting agents include sodium hydroxide, potassium hydroxide, hydrochloric acid, and N-methyl glucamine. The pH of the suspension may be adjusted to about 7.0 to about 11.0. Preferably, the pH is adjusted to about 6.0 to about 8.0. More preferably the pH is adjusted to about one of the following values, i.e., 6.5, 6.6, 6.7, 6.8, 6.9, 7.0, 7.1, 7.2, 7.3, 7.4 or 7.5. The term "about" as used herein in relation to pH means ±0.1 pH units from the specified value.

In certain embodiments, the suspension comprises between 0.1% (wt/wt) and 2% (wt/wt) pimecrolimus (in solid particle form), between 0.01% and 10% of a wetting agent, between 0.01% and 10% of a suspending agent and between 0.01% and 10% of an osmotic agent.

In certain embodiments, the suspension comprises between 0.1% (wt/wt) and 2% (wt/wt) pimecrolimus (in solid particle form), between 0.01% and 1% of a wetting agent, between 0.01% and 1% of a suspending agent and between 0.1% and 10% of an osmotic agent.

In certain embodiments, the suspension comprises between 0.1% (wt/wt) and 2% (wt/wt) pimecrolimus (in solid particle form), between 0.05% and 0.5% of a wetting agent, between 0.05% and 0.5% of a suspending agent and between 1% and 10% of an osmotic agent.

In certain embodiments, the suspension comprises between 0.1% (wt/wt) and 1% (wt/wt) pimecrolimus (in solid particle form), between 0.05% and 0.5% of a wetting agent, between 0.05% and 0.5% of a suspending agent and between 1% and 10% of an osmotic agent.

It is to be understood that the final concentrations of the wetting agent, the suspending agent and/or the osmotic agent may depend on the specific type of agent. The skilled person is, however, capable of identifying appropriate concentrations of the wetting agent, the suspending agent and/or the osmotic agent for use in an ophthalmic composition.

In certain embodiments, the suspension may further comprise suitable amounts of a preservative. It is to be understood that the concentration of the preservative depends on the specific type of preservative. In certain embodiments, the preservative may be added at a concentration ranging from 0.001% to 2%, from 0.001% to 1%, from 0.005% to 0.5%, from 0.01% to 0.1% or 0.015%. In certain embodiments, the preservative may be benzalkonium chloride.

The pH-adjusting agent may be added to the composition at a suitable concentration to reach the desired pH.

In certain embodiments, the composition according to the invention comprises between 0.1% (wt/wt) and 2% (wt/wt) pimecrolimus (in solid particle form), between 0.01% and 1% of a poloxamer (e.g., poloxamer 407), between 0.01% and 1% of a carbomer (e.g., Carbopol 974P) and between 0.1% and 10% of a carbohydrate (e.g., mannitol).

In certain embodiments, the composition according to the invention comprises between 0.1% (wt/wt) and 2% (wt/wt) pimecrolimus (in solid particle form), between 0.05% and 0.5% of a poloxamer (e.g., poloxamer 407), between 0.05% and 0.5% of a carbomer (e.g., Carbopol 974P) and between 1% and 10% of a carbohydrate (e.g., mannitol).

In certain embodiments, the composition according to the invention comprises between 0.1% (wt/wt) and 1% (wt/wt) pimecrolimus (in solid particle form), between 0.05% and 0.5% of a poloxamer (e.g., poloxamer 407), between 0.05% and 0.5% of a carbomer (e.g., Carbopol 974P) and between 1% and 10% of a carbohydrate (e.g., mannitol).

In certain embodiments, the composition according to the invention comprises 0.3% (wt/wt) pimecrolimus (in solid particle form), between 0.05% and 0.5% of a poloxamer (e.g., poloxamer 407), between 0.05% and 0.5% of a carbomer (e.g., Carbopol 974P) and between 1% and 10% of a carbohydrate (e.g., mannitol).

In certain embodiments, the composition according to the invention comprises 0.3% (wt/wt) pimecrolimus (in solid particle form), 0.1% of a poloxamer (e.g., poloxamer 407), 0.2% of a carbomer (e.g., Carbopol 974P) and 4.3% of a carbohydrate (e.g., mannitol).

In certain embodiments, the composition according to the invention comprises 1% (wt/wt) pimecrolimus (in solid particle form), between 0.05% and 0.5% of a poloxamer (e.g., poloxamer 407), between 0.05% and 0.5% of a carbomer (e.g., Carbopol 974P) and between 1% and 10% of a carbohydrate (e.g., mannitol).

In certain embodiments, the composition according to the invention comprises 1% (wt/wt) pimecrolimus (in solid particle form), 0.1% of a poloxamer (e.g., poloxamer 407), 0.2% of a carbomer (e.g., Carbopol 974P) and 4.3% of a carbohydrate (e.g., mannitol).

In certain embodiments, the composition according to the invention comprises 1% (wt/wt) pimecrolimus (in solid particle form), 0.1% of a poloxamer (e.g., poloxamer 407), 0.125% of a carbomer (e.g., Carbopol 974P) and 4.3% of a carbohydrate (e.g., mannitol).

As a preservative, the suspension preferably comprises between 0.001% and 1% benzalkonium chloride, preferably between 0.005 and 0.5% benzalkonium chloride, more preferably between 0.01% and 0.1% benzalkonium chloride, most preferably 0.015% benzalkonium chloride.

Furthermore, the pH of the suspension is preferably adjusted to pH 6.90, even more preferably wherein the pH is adjusted using sodium hydroxide.

In certain embodiments, the suspension may have the following composition:

**Table 1: Suspensions comprising pimecrolimus**

| **Ingredient** | **Concentration** |
|---|---|
| Pimecrolimus (micronized) | 1% (10 mg/mL) |
| Pluronic F127 NF (Poloxamer 407) | 0.1% (1 mg/mL) |
| Carbopol 974P | 0.125% or 0.2% (1.25 or 2 mg/mL) |
| Mannitol | 4.3% (43 mg/ml) |
| Benzalkonium Chloride | 0.015% |
| Sodium Hydroxide | Adjust to pH 6.9 |
| Water | *qs* |

The suspension as defined herein above may be obtained by sequentially dissolving poloxamer 407, mannitol and benzalkonium chloride in water in a first tank. Afterwards, micronized pimecrolimus particles may be slowly added to the first tank and homogenized in a homogenizer set to 4800 RPM for 30 minutes. In a second tank, Carbopol 974P may be mixed in water and homogenized. After the homogenization step, the pH in the second tank may be adjusted with sodium hydroxide to pH 6.5-7.0. To obtain the final suspension, the contents of tanks 1 and 2 may be combined. Afterwards, the suspension may be autoclaved at 121°C for 15 minutes.

In certain embodiments, the suspension may further comprise glycerol.

In certain embodiments, the composition of the invention may be formulated as a solution. The term "solution" as used herein refers to a liquid mixture in which a solute is uniformly distributed within a solvent. The skilled person is capable of formulating an ophthalmologically suitable solution comprising pimecrolimus as the active ingredient.

In certain embodiments, the composition may be formulated as a semi-solid. The term "semi-solid' as used herein refers to a system "having the qualities of both a solid and a liquid" (cf Medical Definition in Marriam-Webster Dictionary) and is suitable for topical administration.

In a particular embodiment, the invention relates to the composition for use according to the invention, wherein the composition is an ointment. The term "ointment", as used herein, refers to a semisolid composition that is thicker than a cream and generally has a higher concentration of oils as compared to a cream. Ointments are not water-soluble due to the amount of oil, hydrocarbons and/or waxes contained therein.

The ointment may have any composition that renders it suitable for ophthalmic applications.

In certain embodiments, the ointment may be any one of the ointments disclosed in WO 2020/234389.

That is, in a particular embodiment, the invention relates to the composition for use according to the invention, wherein the ointment comprises a medium-chain triglyceride and a microcrystalline wax.

That is, the ointment for use according to the invention may comprise any combination of a medium-chain triglyceride and a microcrystalline wax in any amount.

The term "triglyceride" as used herein refers to an ester derived from glycerol and three fatty acids. Medium-chain triglycerides are triglycerides comprising C6 to C12 fatty acids, e.g. as known and commercially available under the trade name Acomed^{®}, Myritol^{®}, Captex^{®}, Neobee^{®}M5F, Miglyol^{®}810, Miglyol^{®}812, Mazol^{®}, Sefsol^{®}860 and Sefsol^{®}870. Such medium-chain triglycerides are usually obtained from the oil extracted from the hard, dried fraction of the endosperm of *Cocos nucifera* or from the dried endosperm of *Elaeis guineensis.* They consist of a mixture of triglycerides of saturated fatty acids. Mainly of caprylic acid and of capric acid, and contain not less than 95 percent of saturated fatty acids with 8 to 10 carbon atoms.

A microcrystalline wax is characterised by its fine crystalline structure in contrast to the larger crystalline structure of paraffin wax. Microcrystalline waxes contain long, branched hydrocarbon chains. Microcrystalline waxes are more flexible, less oily, have higher tensile strength, more adhesion, and higher melting points than paraffin wax and have excellent gelling properties. The composition of the present invention may comprise any microcrystalline wax known in the art. Preferably, the microcrystalline wax comprised in the composition according to the invention follows the US Pharmacopeia (USP 43-NF38) requirements.

The composition of the present invention may comprise any medium-chain triglyceride in any amount that is suitable to obtain the claimed technical effect. In a preferred embodiment, the invention relates to a composition for use according to the invention, wherein the content of the medium-chain triglyceride is between 30% (wt/wt) and 60% (wt/wt). In a more preferred embodiment, the invention relates to a composition for use according to the invention, wherein the content of the medium-chain triglyceride is 50% (wt/wt).

The composition of the present invention may comprise any microcrystalline wax in any amount that is suitable to obtain the claimed technical effect. In a preferred embodiment, the invention relates to a composition for use according to the invention, wherein the content of the microcrystalline wax is between 3% (wt/wt) and 10% (wt/wt). In a more preferred embodiment, the invention relates to a composition according to the invention, wherein the content of the microcrystalline wax is 6% (wt/wt).

In a particular embodiment, the invention relates to a composition for use according to the invention comprising 0.1% (wt/wt) to 2% (wt/wt) pimecrolimus, between 30% and 60% medium-chain triglyceride and between 3% and 10% microcrystalline wax.

In a particular embodiment, the invention relates to a composition for use according to the invention comprising 0.3% (wt/wt) to 1% (wt/wt) pimecrolimus, between 30% and 60% medium-chain triglyceride and between 3% and 10% microcrystalline wax.

In a particular embodiment, the invention relates to a composition for use according to the invention comprising 0.3% pimecrolimus, 50% medium-chain triglyceride and 6% microcrystalline wax.

In another embodiment, the invention relates to a composition for use according to the invention comprising 0.1% pimecrolimus, 50% medium-chain triglyceride and 6% microcrystalline wax.

In another embodiment, the invention relates to a composition for use according to the invention comprising 1% pimecrolimus, 50% medium-chain triglyceride and 6% microcrystalline wax.

The composition according to the invention may comprise any medium-chain triglyceride in the above-mentioned amounts, provided that it is suitable to obtain the claimed technical effect. Preferably, the composition according to the invention comprises medium-chain triglycerides comprising decanoglycerides and/or octanoglycerides. Accordingly, in a preferred embodiment, the invention relates to a composition for use according to the invention, wherein the medium-chain triglyceride comprises caprylic acid and/or capric acid.

That is, the medium-chain triglyceride comprised in the composition according to the invention may comprise one, two or three caprylic acid fatty acid moieties and/or one, two or three capric acid fatty acid moieties. The medium-chain triglyceride comprised in the composition according to the invention may further be a mixture of triglycerides, wherein at least 95% of the triglycerides comprise one, two or three caprylic acid fatty acid moieties and/or one, two or three capric acid fatty acid moieties, respectively.

Caprylic acid is the common name for the eight-carbon saturated fatty acid known by the systematic name octanoic acid. Capric acid is a saturated medium-chain fatty acid with a ten-carbon backbone.

The commercially available medium-chain triglyceride Miglyol^{®}812 comprises caprylic acid and capric acid. Thus, in a more preferred embodiment, the invention relates to a composition for use according to the invention, wherein the medium-chain triglyceride is Miglyol^{®}812. In a particular embodiment, the invention relates to a composition for use according to the invention, wherein the medium chain triglyceride comprises a mixture of triglycerides with 50-64% C8 & 30-45% C10 saturated fatty acids.

The ointment is preferably formulated in an ointment base. Suitable ointment bases include for example ophthalmologically acceptable oil and fat bases. Suitable ointment bases are, without limitation:
(a) natural wax e.g. white and yellow bees wax, carnauba wax, wool wax (wool fat), purified lanolin, anhydrous lanolin,
(b) petroleum wax e.g. hard paraffin, microcrystalline wax,
(c) hydrocarbons e.g. liquid paraffin, white and yellow soft paraffin, white petrolatum, yellow petrolatum, or
(d) combinations thereof.

Within the present invention, the ointment is preferably based on white petrolatum.

In certain embodiments, the ointment may comprise a preservative, in particular any one of the preservatives disclosed herein. In certain embodiments, the ointment may comprise phenyl ethyl alcohol. In certain embodiments, phenyl ethyl alcohol may be present in the ointment at a concentration between 0.5% (wt/wt) and 2% (wt/wt), in particular at a concentration of 1.0% (wt/wt).

In certain embodiments, the composition of the invention is an ointment comprising 0.1%, 0.3% or 1% pimecrolimus (wt/wt), 50% medium-chain triglyceride, 6% microcrystalline wax and an ointment base, wherein the ointment base is preferably white petrolatum.

In other embodiments, the composition of the invention is an ointment comprising 0.1%, 0.3% or 1% pimecrolimus (wt/wt), 50% medium-chain triglyceride, 6% microcrystalline wax, 1% phenyl ethyl alcohol and an ointment base, wherein the ointment base is preferably white petrolatum.

In a further embodiment, the composition of the invention is an ointment consisting of 0.1%, 0.3% or 1% pimecrolimus (wt/wt), 50% medium-chain triglyceride, 6% microcrystalline wax and an ointment base, wherein the ointment base is preferably white petrolatum.

In a further embodiment, the composition of the invention is an ointment consisting of 0.1%, 0.3% or 1% pimecrolimus (wt/wt), 50% medium-chain triglyceride, 6% microcrystalline wax, 1% phenyl ethyl alcohol and an ointment base, wherein the ointment base is preferably white petrolatum.

In certain embodiments, the ointment may have the following compositions:

**Table 2: Ointments comprising pimecrolimus**

| | **Composition 1** | **Composition 2** |
|---|---|---|
| Ingredient | Amount (mg/g) | Amount (mg/g) |
| Pimecrolimus | 3.0 | 10.0 |
| Triglycerides, medium-chain | 500.0 | 500.0 |
| Microcrystalline wax | 60.0 | 60.0 |
| Phenyl ethyl alcohol | 10.0 | 10.0 |
| White Petrolatum | qs to 1.0 g | qs to 1.0 g |
| **Total mass** | **1.0 g** | **1.0 g** |

In certain embodiments, the composition of the invention may be formulated as a cream. The term "cream" as used herein relates to an emulsion of oil and water in approximately equal proportions. The skilled person is capable of formulating an ophthalmologically suitable cream comprising pimecrolimus as the active ingredient.

In certain embodiments, the composition of the invention may be formulated as a gel. The term "gel" as used herein refers to a heterogeneous mixture containing a gelling agent, wherein at least one component is dissolved in a liquid phase. The term "gelling agent," as used herein, refers to a pharmaceutically acceptable excipient known in the art to produce a gel upon mixing with a solvent (e.g., an aqueous solvent). Non-limiting examples of gelling agents include hyaluronan, a polyoxyethylene-polyoxypropylene block copolymer (e.g., a poloxamer), poly(lactic-co-glycolic) acid, polylactic acid, polycaprolactone, alginic acid or a salt thereof, polyethylene glycol, a cellulose, a cellulose ether, a carbomer (e.g., Carbopol), agaragar, gelatin, glucomannan, galactomannan (e.g., guar gum, locust bean gum, or tara gum), xanthan gum, chitosan, pectin, starch, tragacanth, carrageenan, polyvinylpyrrolidone, polyvinyl alcohol, paraffin, petrolatum, silicates, fibroin, and combinations thereof.

In certain embodiments, the composition of the invention may be formulated as a paste. The term "paste" as used herein refers to a semisolid preparation of high viscosity.

The composition of the invention may be delivered to a dry eye patient by any suitable route of administration. It is, however, preferred that the composition of the invention is topically administered to the eye. In certain embodiments, the composition of the invention, in particular the suspension of the invention is administered to the conjunctival sac. The conjunctival sac is the space bound by the conjunctival membrane between the palpebral and bulbar conjunctiva, into which the lacrimal fluid is secreted; it is a closed space when eye is closed; when eye is open, the sac is open anteriorly through the palpebral fissure (between the eyelids).

In certain embodiments, the composition of the invention is administered to the ocular surface. The term "ocular surface", as used herein, refers to the wetted-surface and glandular epithelia of the cornea, conjunctiva, lacrimal gland, accessory lacrimal glands, nasolacrimal duct and meibomian gland, and their apical and basal matrices, puncta and adjacent or related structures, including the eyelids linked as a functional system by both continuity of epithelia, by innervation, and the endocrine and immune systems.

The composition of the invention is preferably administered to the dry eye patient in an effective amount, i.e., an amount sufficient to alleviate the signs and/or symptoms of dry eye in a human and/or animal subject, preferably when administered for a sufficient amount of time. The effective amount may be calculated or estimated based on various factors, such as, without limitation, the concentration of the active agent pimecrolimus in the composition, the effect to be achieved and the pharmacodynamics associated with the active agent in the subject.

The skilled person is able to determine a suitable unit dose of the composition that is to be administered to a subject as well as the frequency with which unit doses are administered. As used herein, the term "unit dose" refers to a single dose of the composition of the invention that is sufficient to produce a desired therapeutic effect.

In certain embodiments, the composition of the invention is a liquid, such as a suspension or solution. The suspension or solution of the invention is preferably administered to the conjunctival sac and/or the ocular surface of an animal and/or human subject in the form of eye drops. In such embodiments, a unit dose may correspond to 1, 2, 3, 4 or 5 drops of the suspension or solution according to the invention. Preferably, a unit dose corresponds to 1 drop of the suspension or solution according to the invention.

In certain embodiments, a semi-solid, such as the ointment or cream of the invention, is administered to the ocular surface of a dry eye patient. In such embodiments, a unit dose may correspond to a 1, 2, 3, 4, 5, 6, 7, 8, 9, or 10 mm strip of the semi-solid of the invention. Preferably, a unit dose corresponds to a 3 mm strip of the semi-solid of the invention, more preferably of the ointment of the invention.

In certain embodiments, a unit dose of the composition of the invention may be packaged in a physically discrete pack - equals a Single Dose Unit (SDU). That is, in certain embodiments, the invention relates to a Single Dose Unit, containing an amount of sterile, non-preserved drug product, as a liquid, ointment, cream, gel or paste, that is sufficient for a single application.

In a particular embodiment, the invention relates to the composition for use according to the invention, wherein a unit dose of the composition is administered once, twice, three times, four times a day.

That is, in certain embodiments, a unit dose is administered to the dry eye patient, preferably to the conjunctival sac and/or the ocular surface of the dry eye patient, once a day. In certain embodiments, a unit dose is administered to the dry eye patient, preferably to the conjunctival sac and/or the ocular surface of the dry eye patient, twice a day. in certain embodiments, a unit dose is administered to the dry eye patient, preferably to the conjunctival sac and/or the ocular surface of the dry eye patient, three times a day. in certain embodiments, a unit dose is administered to the dry eye patient, preferably to the conjunctival sac and/or the ocular surface of the dry eye patient, four times a day.

In a preferred embodiment, a unit dose is administered to the dry eye patient, preferably to the conjunctival sac and/or the ocular surface of the dry eye patient, twice a day. In a particularly preferred embodiment, a unit dose of the suspension according to the invention is administered to the dry eye patient twice daily, preferably in the form of an eye drop.

The composition according to the invention may be administered for any suitable period of time, in particular for as long the signs and symptoms of dry eye persist. In certain embodiments, the composition of the invention may be administered for a period of 2 to 48 weeks, more preferably for a period of 6 to 36 weeks, even more preferably for a period of 12 to 24 weeks. In certain embodiments, the composition according to the invention may be administered for about 6 weeks, for about 12 weeks or for about 24 weeks.

In certain embodiments, the patient suffers from chronic dry eye and the composition of the invention is administered continuously. That is, in certain embodiments, the composition according to the invention may be administered one or more times per day for an unspecified period of time. In certain embodiments, the composition according to the invention may be administered one or more times per week for an unspecified period of time.

In certain embodiments, the patient suffers from chronic dry eye and the composition of the invention is administered as a chronic intermittent treatment.

As used herein, the term "chronic intermittent treatment" refers to repeated treatment with the composition of the invention of a duration wherein the benefit of the treatment is maintained/maximized throughout the duration of the treatment, and treatments are separated by periods of sufficient duration such that repeated treatment does not lessen the benefit of the treatment.

In certain embodiments, the composition according to the invention is administered until a desired treatment effect is achieved. Thus, in a particular embodiment, the invention relates to the composition for use according to the invention, wherein the subjects show at least a 0.2, 0.3, 0.4, 0.5, 0.6 or 0.7 score unit greater improvement (Oxford scale) from baseline at the end of the treatment than with a placebo/vehicle control for the mean score for corneal staining. in a particular embodiment, the invention relates to the composition for use according to the invention, wherein the subjects show at least an at least 3 mm, 4 mm, 5 mm, 6 mm, 7 mm, 8 mm, 9 mm, 10 mm, 11 mm, 12 mm, 13 mm, 14 mm or 15 mm greater improvement (on a 100 mm visual analog scale (VAS)) from baseline at the end of the treatment than with a placebo/vehicle control for the mean score for ocular discomfort.

In certain embodiments, the invention relates to the composition for use according to the invention, wherein the subjects show at least a 0.2 score unit greater improvement (Oxford scale) from baseline at the end of the treatment than with a placebo/vehicle control for the mean score for corneal staining and/or an at least 3 mm greater improvement (visual analog scale (VAS)) from baseline at the end of the treatment than with a placebo/vehicle control for the mean score for ocular discomfort.
in certain embodiments, the invention relates to the composition for use according to the invention, wherein the subjects show at least a 0.3 score unit greater improvement (Oxford scale) from baseline at the end of the treatment than with a placebo/vehicle control for the mean score for corneal staining and/or an at least 4 mm greater improvement (visual analog scale (VAS)) from baseline at the end of the treatment than with a placebo/vehicle control for the mean score for ocular discomfort.
in certain embodiments, the invention relates to the composition for use according to the invention, wherein the subjects show at least a 0.3 score unit greater improvement (Oxford scale) from baseline at the end of the treatment than with a placebo/vehicle control for the mean score for corneal staining and/or an at least 5 mm greater improvement (visual analog scale (VAS)) from baseline at the end of the treatment than with a placebo/vehicle control for the mean score for ocular discomfort.
in certain embodiments, the invention relates to the composition for use according to the invention, wherein the subjects show at least a 0.3 score unit greater improvement (Oxford scale) from baseline at the end of the treatment than with a placebo/vehicle control for the mean score for corneal staining and/or an at least 10 mm greater improvement (visual analog scale (VAS)) from baseline at the end of the treatment than with a placebo/vehicle control for the mean score for ocular discomfort.
in certain embodiments, the invention relates to the composition for use according to the invention, wherein the subjects show at least a 0.4 score unit greater improvement (Oxford scale) from baseline at the end of the treatment than with a placebo/vehicle control for the mean score for corneal staining and/or an at least 10 mm greater improvement (visual analog scale (VAS)) from baseline at the end of the treatment than with a placebo/vehicle control for the mean score for ocular discomfort.
in certain embodiments, the invention relates to the composition for use according to the invention, wherein the subjects show at least a 0.5 score unit greater improvement (Oxford scale) from baseline at the end of the treatment than with a placebo/vehicle control for the mean score for corneal staining and/or an at least 10 mm greater improvement (visual analog scale (VAS)) from baseline at the end of the treatment than with a placebo/vehicle control for the mean score for ocular discomfort.
in certain embodiments, the invention relates to the composition for use according to the invention, wherein the subjects show at least a 0.5 score unit greater improvement (Oxford scale) from baseline at the end of the treatment than with a placebo/vehicle control for the mean score for corneal staining and/or an at least 15 mm greater improvement (visual analog scale (VAS)) from baseline at the end of the treatment than with a placebo/vehicle control for the mean score for ocular discomfort.

In a particular embodiment, the invention relates to the composition for use according to the invention, wherein the subjects show at least a 1.1, 1.2, 1.3, 1.4, 1.5 or 1.6 score unit improvement (Oxford scale) from baseline at the end of the treatment for the mean score for corneal staining. in a particular embodiment, the invention relates to the composition for use according to the invention, wherein the subjects show at least an at least 20 mm, 21 mm, 22 mm, 23 mm, 24 mm, 25 mm, 26 mm, 27 mm, 28 mm, 29 mm or 30 mm improvement (visual analog scale (VAS)) from baseline at the end of the treatment for the mean score for ocular discomfort.

In certain embodiments, the invention relates to the composition for use according to the invention, wherein the subjects show at least a 1.1 score unit improvement (Oxford scale) from baseline at the end of the treatment for the mean score for corneal staining and/or an at least 20 mm improvement (visual analogue scale (VAS)) from baseline at the end of the treatment for the mean score for ocular discomfort.

In certain embodiments, the invention relates to the composition for use according to the invention, wherein the subjects show at least a 1.2 score unit improvement (Oxford scale) from baseline at the end of the treatment for the mean score for corneal staining and/or an at least 22 mm improvement (visual analogue scale (VAS)) from baseline at the end of the treatment for the mean score for ocular discomfort.

In certain embodiments, the invention relates to the composition for use according to the invention, wherein the subjects show at least a 1.3 score unit improvement (Oxford scale) from baseline at the end of the treatment for the mean score for corneal staining and/or an at least 25 mm improvement (visual analogue scale (VAS)) from baseline at the end of the treatment for the mean score for ocular discomfort.

In certain embodiments, the invention relates to the composition for use according to the invention, wherein the subjects show at least a 1.4 score unit improvement (Oxford scale) from baseline at the end of the treatment for the mean score for corneal staining and/or an at least 25 mm improvement (visual analogue scale (VAS)) from baseline at the end of the treatment for the mean score for ocular discomfort.

In certain embodiments, the invention relates to the composition for use according to the invention, wherein the subjects show at least a 1.4 score unit improvement (Oxford scale) from baseline at the end of the treatment for the mean score for corneal staining and/or an at least 30 mm improvement (visual analogue scale (VAS)) from baseline at the end of the treatment for the mean score for ocular discomfort.

The term "about" as used herein means in reasonable vicinity of the stated numerical value, such as plus or minus 10%.

In further embodiments, the present application relates to methods of treating keratoconjunctivitis sicca (dry eye) with a composition comprising pimecrolimus as the active ingredient and uses of a composition comprising pimecrolimus (for the manufacture of a medicament) for the treatment of keratoconjunctivitis sicca (dry eye). However, all definitions and embodiments defined herein above apply *mutatis mutandis,* in particular with respect to the composition of the invention and the definition of keratoconjunctivitis sicca (dry eye).

Thus, in a particular embodiment, the invention relates to a method of treating severe keratoconjunctivitis sicca (dry eye) in a subject in need comprising administering a composition comprising pimecrolimus to the subject in need, in particular wherein keratoconjunctivitis sicca (dry eye) is characterized by a corneal staining score of 4 or 5 on the Oxford grading scale.

In a particular embodiment, the invention relates to a use of a composition comprising pimecrolimus for the manufacture of a medicament for the treatment of keratoconjunctivitis sicca (dry eye), wherein keratoconjunctivitis sicca (dry eye) is characterized by a corneal staining score of 4 or 5 on the Oxford grading scale.

### BRIEF DESCRIPTION OF THE FIGURES

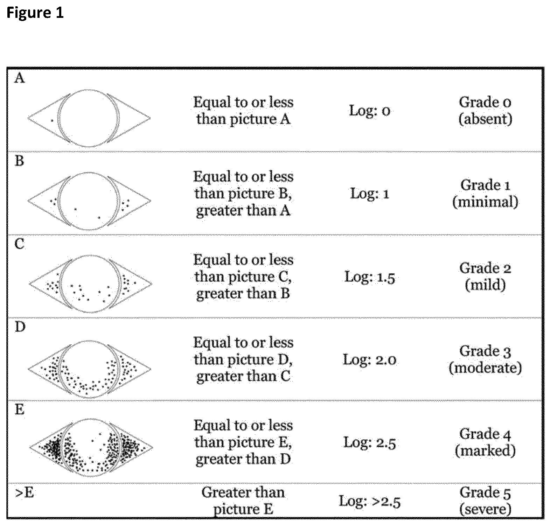

**Figure 1****:** The standard Oxford scale for fluorescein staining.

### EXAMPLES

In the present study, pimecrolimus was evaluated for clinically relevant treatment effects on signs and symptoms of dry eye in patients with moderate to severe KCS.

Primary objective: to demonstrate the efficacy of at least 1 of 2 concentrations of pimecrolimus suspension (1%, or 0.3% or 0.3% and 1%) in patients with moderate to severe KCS by testing the hypothesis that pimecrolimus is superior to placebo/vehicle control in treating the ocular signs and symptoms of dry eye. Week 12 was the primary timepoint.

### 1. Investigational plan

### 1.1 Overall study design and plan

This was a randomized, double-masked, placebo/vehicle-controlled, parallel-group, multi-center study.

The study consisted of 2 periods: a 4 week run-in period on placebo/vehicle and artificial tears (Refresh Plus^{®}) followed by a 24-week double-masked treatment period. The 4 week run-in period on placebo/vehicle b.i.d. and Refresh Plus^{®} p.r.n. ensured randomization of patients (Visit 2) whose condition required drug therapy and reduced the risk for placebo/vehicle responders during the parallel group period of the study. Thereafter, each patient received double-masked treatment in both eyes for 24 weeks. However, only one eye (the eye with the worse corneal staining) was chosen as study eye for efficacy evaluations if both eyes met the eligibility criteria for moderate to severe KCS.

### 1.2 Inclusion criteria

Eligible patients were included who met the following criteria:
1. Patients at least 18 years of age who had given written informed consent.
2. Patients with moderate to severe KCS confirmed by chronic use of ocular lubricants for a minimum of 3 months prior to study start in both eyes. The diagnosis was to have been consistent with tear-deficient rather than evaporative dry eye.
3. Patients were to have the following signs and symptom of dry eye in at least one eye prior to study start:
   - Corneal staining score at least 3 on the Oxford scale 0-5 (see Figure 1 for scale details; score of at least 4 qualifies as severe)
   - Schirmer's test 7 mm or less / 5 min (w/o anesthesia or nasal stimulation). If the Schirmer test results were less than 3mm/5 min the test was to be repeated with nasal stimulation.
   - Ocular discomfort score of at least 40% (moderate) on a visual analog scale (VAS) and a score of at least 2 (moderate) on any one symptom.

An eye was eligible if it met all 3 of the above criteria prior to study start. A patient was eligible if at least one eye was eligible.

### 1.3 Treatments administered

- Pimecrolimus 0.3%
- Pimecrolimus 1.0%
- Placebo/vehicle

Each treatment was to be applied 1 drop b.i.d. at approximately 12-hour intervals into the conjunctival sac.

For both test formulations, the active ingredient was pimecrolimus [0.3% (3 mg/mL) or 1.0% (10 mg/mL)]. The corresponding placebo/vehicle contained 0.125% carbopol.

Use of Refresh Plus^{®} lubricant eyedrops was allowed throughout the study on an as needed (p.r.n.) basis.

### 1.4 Efficacy measurements

The following efficacy assessments were conducted on patient eyes:
Primary efficacy assessments:
- Fluorescein corneal staining (Oxford 0-5 scale)
- Ocular discomfort (VAS,0 -100 mm)

For the purpose of confirmatory hypothesis testing, one sign and one symptom are defined as primary efficacy endpoints together with Week 12 as the primary timepoint.
- absolute change from baseline (at study start/after run-in period) to Week 12 in fluorescein corneal staining (CS) (Oxford Grading Scheme; 0-5 scale)
- absolute change from baseline (at study start/after run-in period) to Week 12 in ocular discomfort (OD) (0-100 mm VAS).

### 2. Analysis of efficacy

The primary efficacy endpoints were change from baseline at Week 12 in corneal staining and change from baseline at Week 12 in ocular discomfort. Due to multiple doses and multiple primary endpoints, a hierarchical testing procedure was used to protect a false positive level of 5%. The Week 24 data were provided for supportive efficacy evidence.

### 2.1 Change from baseline in corneal staining

There was an improvement from baseline in corneal staining (decrease in mean scores) within all treatment groups at all follow-up visits (P ≤ 0.05). The improvement in corneal staining was statistically significant for pimecrolimus 0.3% compared to placebo/vehicle control at Week 12 (P ≤ 0.05) (**Table 3**).

**Table 3: Change from baseline in corneal staining at Weeks 12 and 24 (ITT population)**

| **Visit** | **Statistics** | **Pimecrolimus 0.3% (N=115)** | **Pimecrolimus 1.0% (N=116)** | **Placebo/ vehicle control (N=116)** |
|---|---|---|---|---|
| | **Baseline-Mean** | 3.3 | 3.3 | 3.4 |
| **Week 12 w/LOCF** | Change from Baseline - mean (SE) | -1.3 (0.09) | -1.1 (0.09) | -1.1 (0.09) |
| | Diff. in LS Means vs Placebo/vehicle control | -0.2487 | -0.0714 | |
| | P-value | 0.044* | 0.504 | |
| **Week 24 w/LOCF** | Change from Baseline - mean (SE) | -1.4 (0.11) | -1.3 (0.10) | -1.1 (0.10) |
| | Diff. in LS Means vs Placebo/vehicle control | -0.3062 | -0.1419 | |
| | P-value | 0.059¹ | 0.319 | |

| | | | | |
|---|---|---|---|---|
| *Least square mean differences between treatment groups calculated from ANCOVA with treatment & baseline values in the model* *The p-values from stratified CMH statistics by baseline value* *Missing data at Weeks 12 & 24 imputed using LOCF* **p-value ≤ 0.05 ¹p-value ≤ 0.10* | | | | |

### 2.2 Change from baseline in ocular discomfort

There was an improvement from baseline in ocular discomfort (decrease in mean scores) within all treatment groups at all follow-up visits (P ≤ 0.05). The improvement was greatest with pimecrolimus 1.0% at Week 24 (P = 0.053 compared to Placebo/vehicle control) (**Table 4**).

**Table 4: Change from baseline in ocular discomfort at Weeks 12 and 24 (ITT population)**

| **Visit** | **Statistics** | **Pimecrolimus 0.3% (N=115)** | **Pimecrolimus 1.0% (N=116)** | **Placebo/ vehicle control (N=116)** |
|---|---|---|---|---|
| | **Baseline-Mean** | **62.8** | **61.3** | **63.8** |
| **Week 12 w/LOCF** | Change from Baseline - mean (SE) | -21.5 (2.23) | -20.2 (2.21) | -17.8 (2.33) |
| | Diff. in LS Means vs Placebo/vehicle control | -4.0469 | -3.3177 | |
| | P-value | 0.193 | 0.286 | |
| **Week 24 w/LOCF** | Change from Baseline - mean (SE) | -25.5 (2.31) | -27.1 (2.34) | -21.8 (2.51) |
| | Diff. in LS Means vs Placebo/vehicle control | -4.0999 | -6.443 | |
| | P-value | 0.217 | 0.053¹ | |

| | | | | |
|---|---|---|---|---|
| *The p-values comparing least square mean differences between treatment groups calculated from ANCOVA with treatment & baseline values in the model* *Missing data at Weeks 12 & 24 imputed using LOCF* *¹p-value ≤ 0.10* | | | | |

### 2.3 Subgroup results

Change from baseline in corneal staining and change from baseline in ocular discomfort at Weeks 12 and 24 were analyzed in the pre-specified subgroup of patients with a corneal staining score of 4 or 5 on the Oxford grading scale (equals severe dry eye)

In patients with baseline corneal staining of at least grade 4, there were statistically significant improvements in the 0.3% group compared with placebo/vehicle control for corneal staining and ocular discomfort at Weeks 12 and 24. In the same subgroup of patients, there were statistically significant improvements for pimecrolimus 1.0% compared with placebo/vehicle control for ocular discomfort at Weeks 12 and 24.

In this subgroup of patients with severe KCS, the treatment effects, i.e. the numerical differences relative to placebo/vehicle control, were greater than those observed in the overall study population. This was mainly due to the fact that placebo/vehicle control-treated patients with severe KCS had on average smaller decreases from baseline than the control groups overall (**Tables 5 and 6**).

**Table 5: Change from baseline in corneal staining at Weeks 12 and 24 (Baseline corneal staining grade ≥ 4 - ITT population)**

| **Visit** | **Statistics** | **Pimecrolimus 0.3% (N=34)** | **Pimecrolimus 1.0% (N=28)** | **Placebo/ vehicle control (N=37)** |
|---|---|---|---|---|
| | **Baseline-Mean** | **4.1** | **4.2** | **4.2** |
| **Week 12 w/LOCF** | Change from Baseline - mean (SE) | -1.4 (0.19) | -1.1 (0.22) | -0.8 (0.16) |
| | Diff. in LS Means vs Placebo/vehicle control | -0.5812 | -0.3046 | |
| | P-value | 0.019* | 0.291 | |
| **Week 24 w/LOCF** | Change from Baseline - mean (SE) | -1.6 (0.24) | -1.4 (0.23) | -0.9 (0.19) |
| | Diff. in LS Means vs Placebo/vehicle control | -0.7184 | -0.4380 | |
| | P-value | 0.037* | 0.169 | |

| | | | | |
|---|---|---|---|---|
| *Least square mean differences between treatment groups calculated from ANCOVA with treatment & baseline values in the model* *The p-values from stratified CMH statistics by baseline value* *Missing data at Weeks 12 & 24 imputed using LOCF* **p-value ≤ 0.05* | | | | |

**Table 6: Change from baseline in ocular discomfort at Weeks 12 and 24 (Baseline corneal staining grade ≥ 4 - ITT population)**

| **Visit** | **Statistics** | **Pimecrolimus 0.3% (N=34)** | **Pimecrolimus 1.0% (N=28)** | **Placebo/ vehicle control (N=37)** |
|---|---|---|---|---|
| | **Baseline-Mean** | **67.2** | **62.0** | **64.5** |
| **Week 12 w/LOCF** | Change from Baseline - mean (SE) | -25.6 (4.62) | -23.4 (4.64) | -8.8 (4.22) |
| | Diff. in LS Means vs Placebo/vehicle control | -15.6762 | -15.6319 | |
| | P-value | 0.007* | 0.010* | |
| **Week 24 w/LOCF** | Change from Baseline - mean (SE) | -30.6 (4.78) | -31.6 (5.37) | -13.6 (4.52) |
| | Diff. in LS Means vs Placebo/vehicle control | -15.7914 | -19.006 | |
| | P-value | 0.012* | 0.004* | |

| | | | | |
|---|---|---|---|---|
| *The p-values comparing least square mean differences between treatment groups calculated from ANCOVA with treatment & baseline values in the model* *Missing data at Weeks 12 & 24 imputed using LOCF* **p-value ≤ 0.05* | | | | |

## Claims

1. A composition comprising pimecrolimus for use in the treatment of severe keratoconjunctivitis sicca (dry eye), wherein keratoconjunctivitis sicca (dry eye) is **characterized by** a corneal staining score of 4 or 5 on the Oxford grading scale.

2. The composition for use of claim 1, wherein the composition comprises between 0.1% (wt/wt) and 2% (wt/wt) pimecrolimus, preferably wherein the composition comprises 0.3% (wt/wt) or 1% (wt/wt) pimecrolimus.

3. The composition for use of claim 1 or 2, wherein the composition is a suspension, a solution or a semi-solid, in particular wherein the semi-solid is an ointment a cream, a gel or a paste.

4. The composition for use according to claim 3, wherein the suspension is a water-based suspension, a glycerol-based suspension or a water-glycerol-based suspension.

5. The composition for use according to claim 3 or 4, wherein the suspension further comprises one or more of a wetting agent, a suspending agent, an osmotic agent, a preservative and/or a pH-adjusting agent.

6. The composition according to any one of claims 3 to 5, wherein the suspension comprises a carbomer.

7. The composition for use of claim 3, wherein the ointment comprises a medium-chain triglyceride and a microcrystalline wax.

8. The composition for use of claim 7, wherein the content of the medium-chain triglyceride is between 30% (wt/wt) and 60% (wt/wt), in particular wherein the content of the medium-chain triglyceride is 50% (wt/wt).

9. The composition for use of claim 7 or 8, wherein the content of the microcrystalline wax is between 3% (wt/wt) and 10% (wt/wt), in particular wherein the content of the microcrystalline wax is 6% (wt/wt).

10. The composition for use of any one of claims 7 to 9, wherein the composition is an ointment comprising 0.3% (wt/wt) pimecrolimus, 50% (wt/wt) medium-chain triglyceride and 6% (wt/wt) microcrystalline wax; or
wherein the composition is an ointment comprising 0.1% (wt/wt) pimecrolimus, 50% (wt/wt) medium-chain triglyceride, 6% (wt/wt) microcrystalline wax; or
wherein the composition is an ointment comprising 1% (wt/wt) pimecrolimus, 50% (wt/wt) medium-chain triglyceride, 6% (wt/wt) microcrystalline wax.

11. The composition for use of any one of claims 7 to 10, wherein the medium-chain triglyceride comprises caprylic acid and/or capric acid.

12. The composition for use of any one of claims 7 to 11 further comprising a preservative, in particular wherein the content of the preservative is between 0.5% (wt/wt) and 2% (wt/wt), in particular wherein the content of the preservative is 1.0% (wt/wt).

13. The composition for use claim 12, wherein the preservative is phenyl ethyl alcohol.

14. The composition for use of any one of claims 1 to 13, wherein the treatment period is between 2 weeks and 48 weeks, in particular wherein the treatment period is 12 weeks or 24 weeks.

15. The composition for use of any one of claims 1 to 13, wherein the composition is administered continuously or administered as a chronic intermittent treatment; and/or wherein a unit dose of the composition is administered once, twice, three times, four times a day.

## Patentansprüche

1. Zusammensetzung, die Pimecrolimus umfasst, zur Verwendung bei der Behandlung von schwerer Keratokonjunktivitis sicca (trockenes Auge), wobei Keratokonjunktivitis sicca (trockenes Auge) durch einen Hornhautfärbungswert von 4 oder 5 auf der Oxford-Grading-Skala gekennzeichnet ist.

2. Zusammensetzung zur Verwendung nach Anspruch 1, wobei die Zusammensetzung zwischen 0,1% (Gew./Gew.) und 2% (Gew./Gew.) Pimecrolimus umfasst, vorzugsweise wobei die Zusammensetzung 0,3% (Gew./Gew.) oder 1% (Gew./Gew.) Pimecrolimus umfasst.

3. Zusammensetzung zur Verwendung nach Anspruch 1 oder 2, wobei die Zusammensetzung eine Suspension, eine Lösung oder ein halbfester Stoff ist, insbesondere wobei der halbfeste Stoff eine Salbe, eine Creme, ein Gel oder eine Paste ist.

4. Zusammensetzung zur Verwendung nach Anspruch 3, wobei die Suspension eine Suspension auf Wasserbasis, eine Suspension auf Glycerinbasis oder eine Suspension auf Wasser-Glycerin-Basis ist.

5. Zusammensetzung zur Verwendung nach Anspruch 3 oder 4, wobei die Suspension des Weiteren eines oder mehrere von einem Benetzungsmittel, einem Suspendiermittel, einem osmotischen Mittel, einem Konservierungsmittel und/oder einem Mittel zum Einstellen des pH-Wertes umfasst.

6. Zusammensetzung nach einem der Ansprüche 3 bis 5, wobei die Suspension ein Carbomer umfasst.

7. Zusammensetzung zur Verwendung nach Anspruch 3, wobei die Salbe ein mittelkettiges Triglycerid und ein mikrokristallines Wachs umfasst.

8. Zusammensetzung zur Verwendung nach Anspruch 7, wobei der Gehalt an dem mittelkettigen Triglycerid zwischen 30% (Gew./Gew.) und 60% (Gew./Gew.) beträgt, insbesondere wobei der Gehalt an dem mittelkettigen Triglycerid 50% (Gew./Gew.) beträgt.

9. Zusammensetzung zur Verwendung nach Anspruch 7 oder 8, wobei der Gehalt an dem mikrokristallinen Wachs zwischen 3% (Gew./Gew.) und 10% (Gew./Gew.) beträgt, insbesondere wobei der Gehalt an dem mikrokristallinen Wachs 6% (Gew./Gew.) beträgt.

10. Zusammensetzung zur Verwendung nach einem der Ansprüche 7 bis 9, wobei die Zusammensetzung eine Salbe ist, die 0,3% (Gew./Gew.) Pimecrolimus, 50% (Gew./Gew.) mittelkettiges Triglycerid und 6% (Gew./Gew.) mikrokristallines Wachs umfasst; oder
wobei die Zusammensetzung eine Salbe ist, die 0,1% (Gew./Gew.) Pimecrolimus, 50% (Gew./Gew.) mittelkettiges Triglycerid, 6% (Gew./Gew.) mikrokristallines Wachs umfasst; oder
wobei die Zusammensetzung eine Salbe ist, die 1% (Gew./Gew.) Pimecrolimus, 50% (Gew./Gew.) mittelkettiges Triglycerid, 6% (Gew./Gew.) mikrokristallines Wachs umfasst.

11. Zusammensetzung zur Verwendung nach einem der Ansprüche 7 bis 10, wobei das mittelkettige Triglycerid Caprylsäure und/oder Caprinsäure umfasst.

12. Zusammensetzung zur Verwendung nach einem der Ansprüche 7 bis 11, des Weiteren umfassend ein Konservierungsmittel, insbesondere wobei der Gehalt an dem Konservierungsmittel zwischen 0,5% (Gew./Gew.) und 2% (Gew./Gew.) beträgt, insbesondere wobei der Gehalt an dem Konservierungsmittel 1,0% (Gew./Gew.) beträgt.

13. Zusammensetzung zur Verwendung nach Anspruch 12, wobei das Konservierungsmittel Phenylethylalkohol ist.

14. Zusammensetzung zur Verwendung nach einem der Ansprüche 1 bis 13, wobei der Behandlungszeitraum zwischen 2 Wochen und 48 Wochen beträgt, insbesondere wobei der Behandlungszeitraum 12 Wochen oder 24 Wochen beträgt.

15. Zusammensetzung zur Verwendung nach einem der Ansprüche 1 bis 13, wobei die Zusammensetzung kontinuierlich verabreicht wird oder als eine chronische intermittierende Behandlung verabreicht wird; und/oder wobei eine Einheitsdosis der Zusammensetzung einmal, zweimal, dreimal, viermal täglich verabreicht wird.

## Revendications

1. Composition comprenant du pimécrolimus destinée à être utilisée dans le traitement de la kératoconjonctivite sèche sévère (œil sec), dans laquelle la kératoconjonctivite sèche (œil sec) est **caractérisée par** un score de coloration cornéenne de 4 ou 5 sur l'échelle de classement Oxford.

2. Composition pour utilisation selon la revendication 1, dans laquelle la composition comprend entre 0,1 % (p/p) et 2 % (p/p) de pimécrolimus, de préférence dans laquelle la composition comprend 0,3 % (p/p) ou 1 % (p/p) de pimécrolimus.

3. Composition pour utilisation selon la revendication 1 ou 2, dans laquelle la composition est une suspension, une solution ou un semi-solide, en particulier dans laquelle le semi-solide est un onguent, une crème, un gel ou une pâte.

4. Composition pour une utilisation selon la revendication 3, dans laquelle la suspension est une suspension à base d'eau, une suspension à base de glycérol ou une suspension à base d'eau-glycérol.

5. Composition à utiliser selon la revendication 3 ou 4, dans laquelle la suspension comprend en outre l'un ou plusieurs parmi un agent mouillant, un agent de suspension, un agent osmotique, un conservateur et/ou un agent d'ajustement du pH.

6. Composition selon l'une quelconque des revendications 3 à 5, dans laquelle la suspension comprend un carbomère.

7. Composition pour utilisation selon la revendication 3, dans laquelle l'onguent comprend un triglycéride à chaîne moyenne et une cire microcristalline.

8. Composition pour utilisation selon la revendication 7, dans laquelle la teneur en triglycéride à chaîne moyenne est comprise entre 30 % (p/p) et 60 % (p/p), en particulier dans laquelle la teneur en triglycéride à chaîne moyenne est de 50 % (p/p).

9. Composition pour utilisation selon la revendication 7 ou 8, dans laquelle la teneur en cire microcristalline est comprise entre 3 % (p/p) et 10 % (p/p), en particulier dans laquelle la teneur en cire microcristalline est de 6 % (p/p).

10. Composition pour utilisation selon l'une quelconque des revendications 7 à 9, dans laquelle la composition est un onguent comprenant 0,3 % (p/p) de pimécrolimus, 50 % (p/p) de triglycéride à chaîne moyenne et 6 % (p/p) de cire microcristalline ; ou
dans laquelle la composition est un onguent comprenant 0,1 % (p/p) de pimécrolimus, 50 % (p/p) de triglycéride à chaîne moyenne, 6 % (p/p) de cire microcristalline ; ou
dans laquelle la composition est un onguent comprenant 1 % (p/p) de pimécrolimus, 50 % (p/p) de triglycéride à chaîne moyenne, 6 % (p/p) de cire microcristalline.

11. Composition pour utilisation selon l'une quelconque des revendications 7 à 10, dans laquelle le triglycéride à chaîne moyenne comprend de l'acide caprylique et/ou de l'acide caprique.

12. Composition pour utilisation selon l'une quelconque des revendications 7 à 11, comprenant en outre un conservateur, en particulier dans laquelle la teneur en conservateur est comprise entre 0,5 % (p/p) et 2 % (p/p), en particulier dans laquelle la teneur en conservateur est de 1,0 % (p/p).

13. Composition pour utilisation selon la revendication 12, dans laquelle le conservateur est l'alcool phényléthylique.

14. Composition pour utilisation selon l'une quelconque des revendications 1 à 13, dans laquelle la période de traitement est comprise entre 2 semaines et 48 semaines, en particulier dans laquelle la période de traitement est de 12 semaines ou 24 semaines.

15. Composition pour utilisation selon l'une quelconque des revendications 1 à 13, dans laquelle la composition est administrée en continu ou administrée sous la forme d'un traitement intermittent chronique ; et/ou dans laquelle une dose unitaire de la composition est administrée une fois, deux fois, trois fois, quatre fois par jour.
